(19) Europäisches Patentamt / European Patent Office / Office européen des brevets

(11) **EP 2 918 293 B1**

(12) **FASCICULE DE BREVET EUROPEEN**

(45) Date de publication et mention de la délivrance du brevet:
**30.11.2022 Bulletin 2022/48**

(21) Numéro de dépôt: **15152576.3**

(22) Date de dépôt: **11.07.2012**

(51) Classification Internationale des Brevets (IPC):
**A61L 2/07** *(2006.01)*    **A61L 2/00** *(2006.01)*
**A61Q 19/00** *(2006.01)*    **A61K 8/06** *(2006.01)*

(52) Classification Coopérative des Brevets (CPC):
**A61L 2/0023; A61K 8/064; A61L 2/07;**
**A61Q 19/00;** A61K 2800/10; A61K 2800/805

(54) **ÉMULSION DERMO-COSMÉTIQUE STERILE ET PROCÉDÉ POUR LA FABRICATION D'UNE TELLE EMULSION**

STERILE DERMOKOSMETISCHE EMULSION UND VERFAHREN ZUR HERSTELLUNG EINER SOLCHEN EMULSION

STERILE DERMOCOSMETIC EMULSION AND METHOD FOR MANUFACTURING SUCH AN EMULSION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **11.07.2011 FR 1156288**

(43) Date de publication de la demande:
**16.09.2015 Bulletin 2015/38**

(60) Demande divisionnaire:
**15193567.3 / 3 009 151**

(62) Numéro(s) de document de la (des) demande(s) initiale(s) en application de l'article 76 CBE:
**12733736.8 / 2 731 629**

(73) Titulaire: **Pierre Fabre Dermo-Cosmétique**
**92100 Boulogne-Billancourt (FR)**

(72) Inventeurs:
• **Delaunay, Jean-Claude**
**34800 Clermont L'Herault (FR)**
• **Legendre, Franck**
**81300 Grauhlet (FR)**

(74) Mandataire: **Plasseraud IP**
**66, rue de la Chaussée d'Antin**
**75440 Paris Cedex 09 (FR)**

(56) Documents cités:
**EP-A1- 1 920 756**    **EP-B1- 1 876 905**
**WO-A2-2007/148022**    **JP-A- S5 945 829**
**US-A- 5 234 495**

## Description

L'invention concerne une émulsion cosmétique stérile et un procédé pour la fabrication d'une telle émulsion. Plus généralement l'invention concerne un produit sous la forme d'une émulsion. Un tel produit comprend au moins deux phases non miscibles, l'une des phases, dite phase interne ou dispersée, étant en suspension, dispersée dans l'autre, dite phase externe ou continue. Dans les produits concernés par la présente invention, la phase interne est condensée ou liquide et la phase externe est liquide.

Une telle émulsion est susceptible d'être « cassée » c'est-à-dire de perdre son caractère émulsif par divers mécanismes tels que le crémage, la floculation ou la coalescence. Ces phénomènes, conduisant à la destruction de l'émulsion, sont particulièrement influencés par la taille des particules en suspension et la viscosité de la phase externe. Plus les particules de la phase interne sont fines, de même que plus la phase externe est visqueuse, et plus l'émulsion sera stable, et inversement. La stabilité mesure, en quelque sorte, le temps que met ladite émulsion à perdre son caractère émulsif.

Dans de nombreux domaines, tels que la cosmétique ou le médicament, un produit sous la forme d'une émulsion doit répondre à des normes de conservation vis-à-vis des proliférations microbiennes. Une des méthodes pour assurer cette conservation est d'inclure dans le produit des conservateurs tels que des esters de l'acide para-hydroxybenzoïque, des sorbates ou des esters de glycol. Ces adjuvants ne participent pas aux principes actifs visés pour les préparations qui les contiennent mais peuvent cependant être mal tolérés. Il est par conséquent souhaitable de proposer des produits, notamment dans le domaine dermo-cosmétique, n'utilisant pas de tels adjuvants conservateurs mais qui présentent une durée de conservation et une propreté microbienne adéquates. À cette fin, lesdits produits doivent être stériles. La stérilité est définie par la norme EN 556 et la pharmacopée européenne en vigueur, comme la probabilité d'un micro-organisme de proliférer dans ledit produit. Typiquement, ladite probabilité, pour un produit stérile, est inférieure à $10^{-6}$. Pour les produits visés par l'invention, la demanderesse a déterminé que la stérilisation doit être effectuée selon un procédé dont la valeur stérilisatrice, F0, est égale à 22 minutes. Cette valeur F0, dont la méthode de détermination est définie par la pharmacopée européenne en vigueur, donne un temps, exprimé en minutes, quantifiant l'effet létal de la chaleur humide à 121 °C sur les micro-organismes viables. Un produit stérile au sens de la pharmacopée européenne a été soumis à un procédé de stérilisation dont la valeur stérilisatrice, F0, est au moins égale à 15 minutes. En pratique ledit procédé peut comporter plusieurs étapes de stérilisation, cumulatives, ne faisant pas appel à une stérilisation par la seule chaleur humide mais dont les effets létaux cumulés sont équivalents à cette valeur F0.

L'effet létal est mesuré par rapport à un germe de référence, le géobacillus stéarothermophilus sporulé. Ces bactéries sont particulièrement résistantes et tolérantes à la chaleur. Ainsi, l'effet de stérilisation visé est celui qu'aurait produit sur ces germes, dans le milieu étudié, un traitement thermique de 22 minutes à 121 °C. Ce traitement croît en temps de manière exponentielle avec la baisse de la température en fonction de la nature du micro-organisme dont la destruction est visée par le traitement. La valeur de F0 est ainsi donnée par la formule :

$$F0 = t.10^{(T-121/z)}$$

où :

- t est le temps de traitement exprimé en minutes ;
- z a la dimension d'une température et est défini par la résistance thermique du micro-organisme considéré. La valeur de z est définie expérimentalement en regard d'un paramètre D. D est un temps de réduction décimal et mesure le temps, à une température donnée, ici à 121 °C, pour réduire la concentration du micro-organisme considéré de 90 %. Pour géobacillus stéarothermophilus, D est égal à une minute.

Ainsi, z est la variation de température qui modifie la valeur de D d'un facteur 10, pour géobacillus stéarothermophilus, z est égal à 10 °C, ces facteurs D et z sont fonction du milieu et varient notamment selon le type d'émulsion ;

- T est la température du traitement.

Ainsi, un traitement de valeur stérilisatrice F0 égale à 22, est un traitement de 22 minutes à 121 °C (394 K), ou encore un traitement de 36 secondes à 135 °C (408 K).

Bien évidemment, un tel échauffement, qui réduit considérablement la viscosité de la phase externe de l'émulsion, a un effet destructeur sur celle-ci, de sorte que les traitements de stérilisation thermique de tels produits sont exclus par l'homme du métier, et l'utilisation d'adjuvants ou d'autres procédés de stérilisation sont préférés. Cependant, ces autres procédés présentent également des inconvénients ou ne sont pas applicables :

- la stérilisation par irradiation, est difficile à mettre ne œuvre et présente des incertitudes quant aux produits de dégradation ;
- la stérilisation par membrane filtrante n'est en général pas adaptée aux produits aussi visqueux que ceux utilisés en dermo-cosmétique, et risque, par ailleurs, de retenir les phases internes lorsque celles-ci se trouvent à l'état condensé ou solide. Finalement elle n'autorise pas un processus continu et des débits compatibles avec une production industrielle du produit.

Le document EP-B-2032175 décrit un procédé de stérilisation, dit UHT, ou Ultra Haute Température, adaptable à la stérilisation de produits cosmétiques tels que ceux visés par l'invention. Le procédé et le dispositif décrits dans ce document utilisent un chauffage indirect de l'émulsion qui est conduite en la maintenant sous pression dans des bains chauffants et réfrigérants au travers d'une gaine. Cette gaine assure l'absence de contact du produit avec les bains en question. Ce type de procédé provoque un gradient thermique entre le produit au contact des parois de la gaine et le produit au cœur de l'écoulement, ainsi qu'un gradient thermique entre le début et la fin du passage dans un même bain. Ces caractéristiques rendent difficiles le contrôle de la valeur létal de la stérilisation ainsi opérée. Ainsi, ce procédé ne permet pas de contrôler rigoureusement les temps de chauffage, de maintien éventuel à haute température et de refroidissement. En effet, notamment lorsque le produit à stériliser est très visqueux, ces temps étant contrôlés par le débit du produit dans la gaine, la finesse de ladite gaine rend difficile l'obtention d'un débit adéquat. De plus, l'échauffement et le refroidissement du produit au cours de son trajet dans le gaine modifient sa viscosité à la fois le long de la gaine et dans sa section et rendent complexe le contrôle du pompage dudit produit et la nécessité d'utiliser des pressions élevées. Par ailleurs, le contact avec les parois chaudes de la gaine est susceptible de dégrader le produit, alors que le contact avec les parois froides de ladite gaine, dans la zone de refroidissement, tend à figer les corps gras contenus dans ledit produit sans possibilité de réhomogénéisation ultérieure. Le contrôle de ce procédé est donc délicat et les résultats peu reproductibles. Finalement, ce procédé n'est pas adapté à un traitement industriel de volumes de produit élevés, à des débits supérieurs à 1 m³/heure.

Les autres procédés de traitement à ultra-haute température connus de l'art antérieur, notamment les procédés par infusion ou par injection appliqués aux produits alimentaires, sont également peu adaptés à la mise en œuvre de ce type de produit et nécessitent, pour une pleine efficacité, que le contact avec la vapeur soit réalisé alors que le produit se trouve en phase liquide. Bien que les qualités de stérilisation requises pour les produits alimentaires soient moins sévères que pour les produits visés par l'invention et que les qualités organoleptiques des produits alimentaires traités par des procédés de stérilisation à ultra haute température soient moins fragiles que la stabilité des émulsions visées par l'invention, il est courant, selon l'art antérieur d'ajouter auxdits produits alimentaires des additifs tensio-actifs pour en renforcer la stabilité vis-à-vis du cycle de stérilisation. Ainsi, le document EP 0524751 et le document JP 2004 105181 décrivent des procédés de stérilisation d'une émulsion alimentaire, laquelle émulsion est préalablement stabilisée par un l'ajout d'un ester. Ce type d'additif tensio-actif est similaire aux agents conservateurs dont l'absence est visée par l'invention. Les documents suivants sont aussi connus dans la domaine : WO 2007/148022, EP 1 876 905, JP S59 45829.

L'invention concerne une émulsion dermo-cosmétique sans adjuvants conservateurs, laquelle émulsion est stérile selon la norme EN 556 et la pharmacopée européenne, et plus précisément avec une stérilité telle qu'obtenue pour une valeur stérilisatrice $F_0 = 22$ minutes vis-à-vis de géobacillus stéarothermophilus sporulé. Une telle émulsion, grâce à son niveau de stérilisation, hors d'atteinte des procédés connus de l'art antérieur, présente une qualité de conservation exceptionnelle sans avoir recours à l'usage d'adjuvants conservateurs, ce qui permet d'améliorer sa tolérance et permet également l'utilisation de principes actifs non compatibles avec ces adjuvants.

L'invention concerne également un procédé en continu pour la fabrication d'une telle émulsion, lequel procédé comprend les étapes consistant à :

[a]. préchauffer (120) ladite émulsion à une vitesse de préchauffage étant comprise entre 0,1 °C.S -1 et 1 °C.S -1 jusqu'à une température de préchauffage, T1, température limite de stabilité de ladite émulsion ;

[b]. réaliser une stérilisation (130), à une valeur stérilisatrice de $F_0 = 22$ minutes, par un procédé à ultrahaute température par infusion de l'émulsion ainsi préchauffée, lequel procédé comprend :

i. un chauffage (131) à une température, T2, de stérilisation, en pulvérisant un jet d'émulsion préchauffée dans une enceinte de stérilisation remplie de vapeur d'eau ;
ii. un maintien (132) durant un temps t à la température de stérilisation ;
iii. un refroidissement (133) sous vide à une température, T3, de fin de stérilisation, dans lequel la température T3 est inférieure à la température T1 et l'écart entre T1 et T3 est inférieur ou égal à 5 K ;

[c]. refroidir (150) l'émulsion depuis la température T3, sous agitation jusqu'à une température, T4, de stockage, la vitesse de refroidissement étant comprise entre 0,01 °C.S -1 et 0,5 °C.s-'.

Ainsi, le procédé objet de l'invention comprend des phases de chauffage et surtout de refroidissement progressif dans le déroulement du processus, de part et d'autre de l'opération de stérilisation. Ces phases de chauffage et de refroidissement progressifs permettent :

- au chauffage, d'amener l'émulsion dans un état de fluidité adapté au procédé de stérilisation par infusion mais sans casser ladite émulsion ;
- au refroidissement de redonner au produit sa qualité d'émulsion qui a été partiellement dégradée au cours de l'opération de stérilisation.

Les vitesses de chauffage et de refroidissement des étapes i) et iii) de la phase de stérilisation par infusion sont imposées par la thermodynamique du procédé.

L'invention est exposée ci-après selon ses modes de réalisation préférés, nullement limitatifs, et en référence aux figures à 1 à 3 dans lesquelles :

- la figure 1 présente un synoptique de la mise en œuvre d'un procédé selon un exemple de réalisation de l'invention ;
- la figure 2 est un exemple de cycle thermique et d'évolution corrélative de la viscosité d'un produit faisant l'objet d'un traitement selon un exemple de réalisation de l'invention ;
- et la figure 3, est un exemple d'évolution en température de la viscosité d'une émulsion pour la détermination de la température limite de stabilité, figure 3A à une température inférieure à cette température, figure 3B à cette température et figure 3C pour une température supérieure à cette température limite de stabilité.

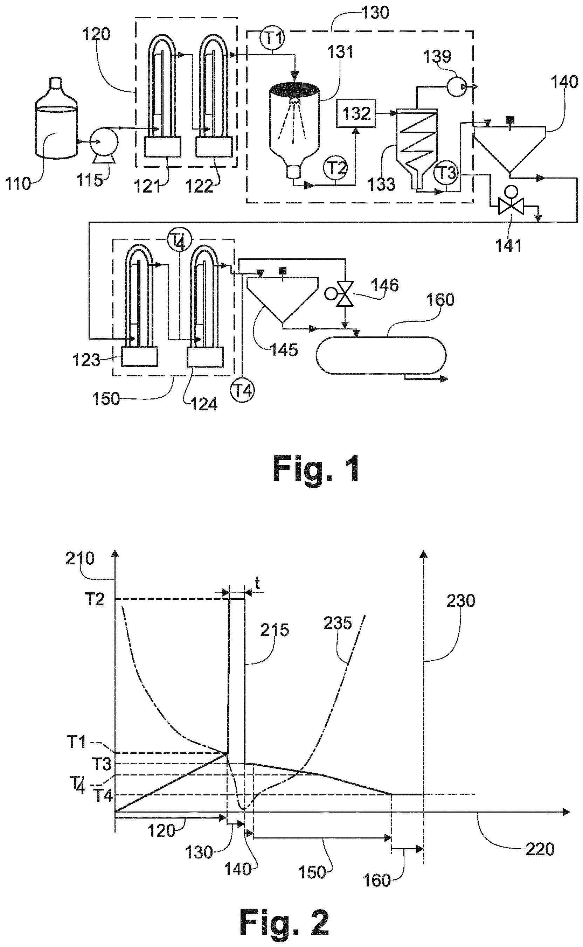

Figure 1, les repères désignent autant les étapes du procédé que les moyens de mise en œuvre de ce procédé au cours desdites étapes. Le produit à stériliser est initialement stocké sous forme d'émulsion dans un réservoir (110). Les produits traités sont principalement des émulsions ayant une phase externe aqueuse et une phase interne grasse. À titre d'exemple, non limitatif, le procédé objet de l'invention est adapté à la stérilisation et à l'obtention d'une émulsion stérile, ladite émulsion comprenant une phase externe, ou continue, aqueuse, comprenant des composés hydrophiles, notamment un gel de carboxymethylcellulose et une phase interne comprenant des matières huileuses et des polyacrylates lipophiles. Le procédé est néanmoins applicable à une émulsion dont la phase continue est grasse et la phase dispersée est aqueuse. Le dispositif et le procédé objets de l'invention peuvent être mis en œuvre pour une large variété d'émulsions, dans des viscosités allant de 600 Cps à 45 000 Cps, c'est-à-dire pour des produits dermo-cosmétiques allant du lait au baume.

De telles émulsions sont obtenues en séparant et en brisant les gouttes de la phase interne de sorte à créer une dispersion homogène de celles-ci dans la phase externe. Cette dispersion est obtenue par l'intermédiaire d'un effet mécanique, l'énergie mécanique ainsi introduite, par agitation ou injection, dans le produit, est stockée dans la tension superficielle des gouttes aux interphases. Le produit est ainsi homogène lorsque la taille des gouttes est sensiblement la même dans toute l'émulsion. Partant de cet état émulsionné, l'émulsion est cassée lorsque, par divers mécanismes, les gouttes de phase interne s'agglomèrent jusqu'à former à nouveau deux phases séparées. Dans ce cas, l'émulsion ne peut être formée qu'en réintroduisant l'énergie mécanique comme initialement. Entre l'émulsion initiale et l'émulsion cassée, il existe des états intermédiaires pour lesquels la répartition de la taille des gouttes de phase interne n'est pas homogène, mais où, sous des conditions thermodynamiques définies ou avec une introduction minimale d'énergie mécanique, l'émulsion peut être à nouveau homogénéisée. Le procédé et le dispositif objets de l'invention visent à conserver à tout moment, et notamment lors des phases les plus sévères du traitement, l'émulsion dans un état qui permette d'obtenir sa réhomogénéisation facile. Ce principe de traitement n'est pas connu des procédés de stérilisation des produits alimentaires émulsionnés tels que le lait.

La figure 2, donnant la température (210) et la viscosité (230) de l'émulsion en fonction du temps (220) permet de suivre le cycle thermique (215) corrélativement à l'évolution de la viscosité (235) de l'émulsion au cours des différentes étapes (120, 130, 140, 160) du procédé objet de l'invention.

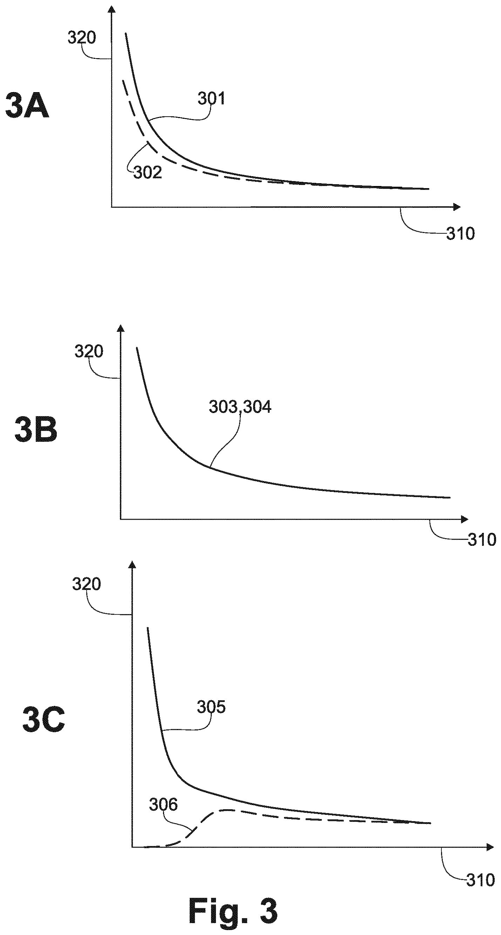

En revenant à la figure 1, des moyens de pompage (115) permettent d'amener le produit à traiter vers deux échangeurs (121, 122) à parois raclées pour porter ledit produit, au cours d'une étape de préchauffage (120), à une température (T1) à la limite de stabilité de l'émulsion. La température T1 est déterminée en analysant l'évolution de la viscosité de l'émulsion en fonction de la température, tel que le représente la figure 3.

Figure 3, l'analyse de l'évolution de la viscosité (320) dynamique d'une émulsion en fonction de son taux de cisaillement (310) est donnée pour des taux de cisaillement, ou gradients de vitesses de cisaillement, croissants (301, 303, 305), ou courbe « aller », et pour des taux de cisaillement décroissants (302, 304, 306), ou courbe « retour ». Cette analyse, réalisée au moyen d'un rhéomètre, comprenant un mobile (cylindre ou cône) cisaillant l'émulsion entre la paroi dudit mobile et une paroi fixe, est effectuée à différentes températures. Ladite analyse fait apparaître trois comportements caractéristiques. Selon l'exemple de réalisation présenté figure 3, le taux de cisaillement (310) varie entre $10 \text{ s}^{-1}$ et $150 \text{ s}^{-1}$.

Figure 3A, pour des températures inférieures à la température T1 la courbe retour (302) fait apparaître des valeurs de viscosité plus faibles que la courbe aller (301) pour un même taux de cisaillement, car l'agitation du mobile tend à fluidifier l'émulsion.

Figure 3C, pour des températures supérieures à T1, l'agitation combinée à la température à l'aller (305) détruit l'émulsion, ce qui est visible sur la courbe retour (306) où la viscosité chute.

Figure 3B, lorsque l'essai est pratiqué à la température T1 la viscosité varie en fonction du taux de cisaillement à l'aller (303) mais redevient de même viscosité après agitation du mobile au retour (304).

Ainsi la température T1 peut être déterminée par l'étude des comportements rhéologiques de l'émulsion en fonction de la température.

En revenant à la figure 1, l'étape de préchauffage (120) utilise avantageusement des échangeurs à parois raclées pour assurer à la fois une montée très progressive et homogène en température de l'émulsion. Ainsi, la vitesse de préchauffage est comprise entre 0,1 °C.s$^{-1}$ et 1 °C.s$^{-1}$.

Le nombre d'échangeurs est choisi en fonction du débit visé, pour permettre un traitement continu, adapté à une utilisation industrielle. Ce débit est notamment supérieur à 1 m$^3$/heure. Un tel échangeur thermique comprend un stator et un rotor pourvu de pâles. Le stator comprend une double paroi chauffée à la température désirée par une circulation fluide, de sorte que la paroi interne de l'échangeur (121, 122) se trouve à la température de consigne T1. Selon un exemple de réalisation avantageux, la température T1 est choisie à 328 K (55 °C). Cette température permet d'obtenir la fluidité requise de l'émulsion sans dégrader celle-ci. Les pâles du rotor raclent en permanence la paroi interne de l'échangeur, de sorte que le temps de contact instantané d'un volume d'émulsion avec ladite paroi est réduit ce qui évite les chocs thermiques pouvant se produire au contact du produit froid avec la paroi chaude de l'échangeur.

L'étape (120) de préchauffage progressif est suivie de l'étape de stérilisation (130). La stérilisation est réalisée selon un procédé dit par infusion. Ce procédé, consiste à pulvériser un jet du produit dans une enceinte remplie de vapeur d'eau à la température désirée. Pour réaliser cette pulvérisation, l'émulsion doit être quasiment liquide. La surface d'échange des gouttes de produit avec la vapeur étant très élevée, le chauffage du produit à la température de stérilisation T2 est quasiment instantané dans tout le volume de produit injecté dans la chambre de stérilisation. Au cours de cet échauffement, le produit absorbe de l'eau correspondant à la condensation de la quantité de vapeur ayant transmis sa chaleur au produit. Le produit est maintenu, au cours d'une étape de maintien (132), à la température T2 puis est dirigé vers un dispositif de refroidissement rapide (133). Avantageusement, T2 est choisie égale à 418 K (145 °C) le temps de maintien, t, lors de l'étape suivante (132) étant de 6 secondes. Ces conditions permettent d'obtenir une valeur stérilisatrice F0 égale à 22 minutes.

La stérilisation (130) se poursuit par un refroidissement rapide (133) dit flash. Le produit, chauffé à la température T2, est mis en communication avec une chambre (133) mise sous vide par des moyens appropriés (139). Le produit aspiré dans cette chambre subit une détente brusque accompagnée d'une libération violente de vapeur. La chaleur latente de vaporisation prélève de l'énergie thermique au produit et ainsi le refroidit, avec arrachement de gouttelettes. Le cycle thermodynamique est réglé de sorte que l'eau absorbée lors du chauffage dans la première phase (131) du cycle de stérilisation est récupérée sous forme de condensât lors du cycle (133) de refroidissement flash. Ainsi, les températures T1 et T3 sont nécessairement proches et l'écart entre T3 et T1 est adapté en fonction de la nature du produit, en fonction de la tenue et de l'aspect de l'émulsion. Le dispositif objet de l'invention devant être apte à traiter différents types de produits, les températures de traitement T1, T2 et T3 sont optimisées pour remplir tous les impératifs à la fois de stérilité, par la valeur de T2 et le temps de maintien, en fonction de la capacité de pouvoir ré-homogénéiser l'émulsion après le traitement de stérilisation notamment par le choix de T1 et de T3. Ces paramètres sont alors figés pour tous les types de produits devant être traités. Ainsi, la demanderesse a déterminé une température T3, avantageuse, égale à 323 K (50 °C). L'ajustement du traitement au produit spécifique est réalisé lors des phases ultérieures (140, 150).

À l'issue de l'étape de stérilisation (130) et selon la nature du produit traité, une étape d'homogénéisation (140) par exemple à l'aide un malaxeur à disques est réalisée. Cette étape vise à ré-homogénéiser rapidement l'émulsion et stopper tout phénomène de coalescence. Alternativement, une vanne (141), en dérivation, permet de sauter cette étape d'homogénéisation pour les produits les plus stables.

Une étape (150) de refroidissement progressif, au moyen d'une pluralité d'échangeurs (123, 124) à parois raclées, permet de ramener l'émulsion à une température (T4) proche ou légèrement supérieure à l'ambiante pour réaliser le stockage de l'émulsion en vue de son conditionnement ultérieur. La vitesse de refroidissement au cours de cette étape de refroidissement progressif est comprise entre 0,01 °C.s$^{-1}$ et 0,5 °C.s$^{-1}$.

Avantageusement, T4 est choisie à 303 K (30 °C), les émulsions étant très stables à cette température, mais suffisamment fluides pour un conditionnement facile. Cette température de stockage en cuve permet également d'éviter les phénomènes de condensation de l'émulsion à l'intérieur de ladite cuve. Le nombre d'échangeurs dans chacun de ces ensembles est choisi en fonction du débit visé.

Le refroidissement est réalisé en plusieurs étapes en respectant la condition d'un écart maximum de 15 K (15 °C) entre deux paliers successifs. Cette caractéristique évite que les gouttes grasses de phase interne de l'émulsion ne se figent au contact d'une paroi trop froide. Si un tel figeage se produisait lesdites gouttes deviendraient impossibles à diviser et à disperser dans la phase externe. Ainsi, en partant de T3 = 323 K (50 °C) pour arriver à T4 = 303 K (30 °C), le refroidissement (150) est réalisé en un minimum de deux étapes. Ainsi, le dispositif objet de l'invention, comprend au moins deux échangeurs correspondant à deux paliers de température. Le premier échangeur (123), ou ensembles d'échangeurs, à parois raclées est réglé à une température T4 juste inférieure à la température T3. Ainsi, pour une température T3 de 323 K (50 °C), T'$_4$ est choisie égale à 313 K (40 °C) et le deuxième échangeur est réglé à la température T4 = 303 K (30 °C). L'utilisation d'échangeurs (123, 124) à parois raclées permet, ici aussi, éviter les chocs thermiques, mais procure également pour certains produits une agitation suffisante pour ré-homogénéiser l'émulsion. Le nombre pratique d'échangeurs est choisi en fonction du débit à atteindre. Ainsi deux groupes d'échangeurs correspondants à ces deux paliers peuvent être installés en parallèle ou plusieurs échangeurs correspondant à des paliers thermiques plus rapprochés peuvent être installés en série.

Selon la nature du produit traité, un ultime malaxage (145), à titre d'exemple dans un malaxeur à disque, permet de finir l'homogénéisation de l'émulsion. Alternativement, pour d'autres produits, cette étape peut être omise, grâce à une vanne (146) placée en dérivation et connectant directement la sortie du deuxième échangeur thermique (124) au réservoir tampon (160) stérile, avant le conditionnement du produit.

Selon un premier exemple de réalisation, le procédé objet de l'invention est adapté à la stérilisation UHT d'un produit consistant en une émulsion dont la phase continue est aqueuse et la phase interne grasse, par exemple un produit comprenant :

- de l'eau (Aqua),
- une huile minérale telle que de l'huile de paraffine (paraffinum liquidum),
- de la glycérine,
- du stéarate de glycéryle,
- de la squalane,
- un carbomer,
- et de la triéthanolamine.

Le procédé objet de l'invention permet de stériliser ce produit avec une valeur stérilisatrice F0 de 22 minutes.

Selon un deuxième exemple de réalisation, le procédé objet de l'invention est adapté à la stérilisation d'un produit dermo-cosmétique comprenant 5 % à 50 % d'une phase continue huileuse et une phase interne aqueuse, par exemple, un produit comprenant :

- de la glycérine,
- de la gomme de xanthane,
- du Steardimonium(Di)Hectorite,
- des triglycérides caprylique/caprique,
- de la cire d'abeille blanche,
- de l'huile d'onagre,
- d'isopropyl palmitate,
- du PEG-30 Dipolyhydroxystearate,
- d'acide citrique monohydraté,
- et de l'eau

Le procédé objet de l'invention permet de stériliser ce produit avec une valeur stérilisatrice F0 de 22 minutes.

Selon un troisième exemple de réalisation, le procédé objet de l'invention est adapté à la stérilisation UHT, d'un produit dermo-cosmétique consistant en un milieu tensio-actif avec association binaire ou ternaire d'anionique amphotère et non ionique, par exemple un produit comprenant :

- du sulfate de coceth de zinc,
- du lauteth disodique sulfosuccinate,
- du polysorbate 20,
- du Ceteareth-60 myristyl glycol,
- de l'acide lactique,
- de l'hydroxyde de sodium,
- et de l'eau.

Le procédé objet de l'invention permet de stériliser ce produit avec une valeur stérilisatrice F0 de 22 minutes.

La description ci avant et les exemples de réalisation montrent clairement que l'invention atteint les objectifs visés. En particulier, elle permet de réaliser un traitement de stérilisation poussée et en continu d'un produit dermo-cosmétique ou d'une préparation galénique fragile, pour les amener à un niveau d'assurance stérilité permettant une longue conservation dudit produit sans adjonction de conservateurs.

## Revendications

1. Émulsion dermo-cosmétique sans adjuvants conservateurs, **caractérisée en ce qu'**elle est stérile selon la norme EN 556 et la pharmacopée européenne, avec un niveau de stérilité tel qu'obtenu pour une valeur stérilisatrice F0 = 22 minutes vis-à-vis de géobacillus stéarothermophilus sporulé.

2. Émulsion selon la revendication 1, comprenant une phase externe aqueuse et une phase interne grasse.

3. Procédé de traitement d'une émulsion en continu pour obtenir une émulsion stérile selon la revendication 1, **caractérisé en ce qu'**il comprend les étapes consistant à :

   [a]. préchauffer (120) ladite émulsion à une vitesse de préchauffage étant comprise entre 0,1 °C.s$^{-1}$ et 1 °C.s$^{-1}$ jusqu'à une température de préchauffage, T1, température limite de stabilité de ladite émulsion ;
   [b]. réaliser une stérilisation (130), à une valeur stérilisatrice de F0 = 22 minutes, par un procédé à ultra-haute température par infusion de l'émulsion ainsi préchauffée, lequel procédé comprend :

   i. un chauffage (131) à une température, T2, de stérilisation, en pulvérisant un jet d'émulsion préchauffée dans une enceinte de stérilisation remplie de vapeur d'eau ;
   ii. un maintien (132) durant un temps t à la température de stérilisation ;
   iii. un refroidissement (133) sous vide à une température, T3, de fin de stérilisation, dans lequel la température T3 est inférieure à la température T1 et l'écart entre T1 et T3 est inférieur ou égal à 5 K ;

[c]. refroidir (150) l'émulsion depuis la température T3, sous agitation jusqu'à une température, T4, de stockage, la vitesse de refroidissement étant comprise entre 0,01 °C.s⁻¹ et 0,5 °C.s⁻¹.

4. Procédé selon la revendication 3, **caractérisé en ce que** l'étape c) de refroidissement (150) est réalisé par paliers, l'écart de température entre deux paliers étant au plus de 15 K (15 °C).

5. Procédé selon la revendication 4, **caractérisé en ce que** la température T2 est de 418 K (145 °C) et *t* = 6 secondes.

6. Procédé selon la revendication 4, pour le traitement d'une émulsion, dans lequel la température T1 est déterminée par un procédé consistant à :

- soumettre l'émulsion à des taux de cisaillement croissants puis décroissants à différentes températures d'essai dans un rhéomètre ;
- tracer l'évolution de la viscosité de l'émulsion en fonction du taux de cisaillement croissant pour obtenir une courbe dite « courbe aller » (301, 303, 305) et l'évolution de la viscosité de l'émulsion en fonction du taux de cisaillement décroissant pour obtenir une courbe dite « courbe retour » (302, 304, 306) ;
- La température T1 étant choisie comme la température d'essai à laquelle la courbe aller (303) et la courbe retour (304) se superposent.

7. Procédé selon la revendication 7 dans lequel le taux de cisaillement varie au cours de l'essai de détermination de T1 entre 10 s⁻¹ et 150 s⁻¹.

8. Procédé selon la revendication 7, **caractérisé en ce que** T1 = 328 K (55 °C).

**Patentansprüche**

1. Dermo-kosmetische Emulsion ohne konservierende Zusatzstoffe, **dadurch gekennzeichnet, dass** sie gemäß der Norm EN 556 und der Europäischen Pharmakopöe steril ist, mit einem Sterilitätsniveau, wie es für einen Sterilisationswert F0 = 22 Minuten gegenüber sporenbildendem Geobacillus stearothermophilus erhalten wird.

2. Emulsion nach Anspruch 1, umfassend eine externe wässrige Phase und eine interne fettige Phase.

3. Verfahren zur kontinuierlichen Behandlung einer Emulsion, um eine sterile Emulsion nach Anspruch 1 zu erhalten, **dadurch gekennzeichnet, dass** es die folgenden Schritte umfasst:

[a]. Vorwärmen (120) der Emulsion mit einer Vorwärmgeschwindigkeit von 0,1 °C.s⁻¹ bis 1 °C.s⁻¹ auf eine Vorwärmtemperatur T1, der Grenztemperatur der Stabilität der Emulsion;
[b]. Durchführen einer Sterilisation (130) bei einem Sterilisationswert von F0 = 22 Minuten durch ein Ultrahochtemperaturverfahren durch Infusion der so vorgewärmten Emulsion, wobei das Verfahren umfasst:

i. Erhitzen (131) auf eine Sterilisationstemperatur T2, indem ein vorgewärmter Emulsionsstrahl in eine mit Wasserdampf gefüllte Sterilisationskammer gesprüht wird;
ii. Halten (132) auf der Sterilisationstemperatur während einer Zeit t;
iii. Abkühlen (133) unter Vakuum auf eine Sterilisationsendtemperatur T3, wobei die Temperatur T3 niedriger als die Temperatur T1 ist und die Differenz zwischen T1 und T3 kleiner oder gleich 5 K ist;

[c]. Abkühlen (150) der Emulsion unter Rühren von der Temperatur T3 auf eine Lagertemperatur T4, wobei die Abkühlungsgeschwindigkeit zwischen 0,01 °C.s⁻¹ und 0,5 °C.s⁻¹ liegt.

4. Verfahren nach Anspruch 3, **dadurch gekennzeichnet, dass** der Schritt c) des Abkühlens (150) in Stufen erfolgt, wobei die Temperaturdifferenz zwischen zwei Stufen höchstens 15 K (15 °C) beträgt.

5. Verfahren nach Anspruch 4, **dadurch gekennzeichnet, dass** die Temperatur T2 418 K (145 °C) beträgt und t=6 Sekunden.

6. Verfahren nach Anspruch 4 zur Behandlung einer Emulsion, wobei die Temperatur T1 durch ein Verfahren bestimmt wird, umfassend:

- Anwenden von steigenden und dann fallenden Scherraten auf die Emulsion bei verschiedenen Prüftemperaturen in einem Rheometer;
- Verfolgen der Viskositätsänderung der Emulsion in Abhängigkeit von der steigenden Scherrate zum Erhalt einer "Vorlaufkurve" (301, 303, 305) und Verfolgen der Viskositätsänderung der Emulsion in Abhängigkeit von der fallenden Scherrate zum Erhalt einer "Rücklaufkurve" (302, 304, 306);
- wobei die Temperatur T1 als Prüftemperatur gewählt wird, bei der sich die Vorlaufkurve (303) und die Rucklaufkurve (304) überlagern.

7. Verfahren nach Anspruch 7, wobei die Scherrate während des Versuchs zur Bestimmung von T1 zwischen 10 s$^{-1}$ und 150 s$^{-1}$ variiert.

8.  Verfahren nach Anspruch 7, **dadurch gekennzeichnet, dass** T1 = 328 K (55 °C).

## Claims

1.  Dermocosmetic emulsion without preservative additives, **characterized in that** it is sterile according to standard EN 556 and the European Pharmacopoeia, with a sterility assurance level such as that achieved for a sterilizing value F0=22 minutes in reference to sporulated geobacillus stearothermophilus.

2.  Emulsion according to claim 1, **characterized in that** it comprises an aqueous external phase and a fatty internal phase.

3.  Method for continuous treating of an emulsion in order to obtain a sterile emulsion according to claim 1, **characterized in that** it comprises the steps consisting of:

    a. pre-heating (120) said emulsion at a pre-heating speed between 0.1°C.s$^{-1}$ and 1° C.s$^{-1}$ up to a pre-heating temperature T1, which temperature is at the stability limit of said emulsion;
    b. performing a sterilization (130) at a sterilizing value F0=22 minutes through a process at ultra-high temperature by infusion of said emulsion, thus pre-heated, which process comprises:

      i. heating (131) up to a temperature T2, of sterilization, by pulverizing a jet of pre-heated emulsion into an enclosed sterilization space filled with water steam ;
      ii. plateauing (132) during a time t at the sterilization temperature;
      iii. cooling (133) under vacuum at an end-of-sterilization temperature T3; wherein temperature T3 is lower than temperature T1, with the difference between T1 and T3 is less than or equal to 5 K ;

    c. cooling (150) the emulsion under agitation from temperature T3 to a storage temperature, T4, the cooling speed being between 0.01°C.s$^{-1}$ and 0.5° C.s$^{-1}$.

4.  Method according to claim 3, **characterized in that** the cooling step c) (150) is performed in plateaus, with a change in temperature between two plateaus of 15K (15°C) at most.

5.  Method according to claim 4, **characterized in that** temperature T2 is 418 K (145°C) and $t$ = 6 seconds.

6.  Method according to claim 4, for treating an emulsion, **characterized in that** temperature T1 is determined by a test consisting of :

    - submitting the emulsion at increasing shearing rates, then decreasing shearing rates, at various test temperatures within a rheometer;
    - tracing the emulsion's viscosity evolution depending on the increasing shearing rate to obtain a curve called "outward curve" ( 301, 303, 305 ) and the emulsion's viscosity evolution depending on the decreasing shearing rate to obtain a curve called "return curve" (302, 304, 306);
    - the temperature T1 being chosen as the test temperature at which the outward curve (303) and the return curve (304) superimpose.

7.  Method according to claim 6, **characterized in that** the shearing rate varies during the test for determining T1 between 10 s$^{-1}$ and 150 s$^{-1}$.

8.  Method according to claim 7, **characterized in that** T1 = 328K (55°C).

# Fig. 1

# Fig. 2

**3A**

320

301

302

310

**3B**

320

303,304

310

**3C**

320

305

306

310

**Fig. 3**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2032175 B
- EP 0524751 A
- JP 2004105181 A
- WO 2007148022 A
- EP 1876905 A
- JP 59045829 A